# EUROPEAN PATENT SPECIFICATION

(11) **EP 1 485 357 B1**
(45) Date of publication and mention of the grant of the patent: **06.07.2005**
(21) Application number: 03744359.5
(22) Date of filing: 13.03.2003
(51) Int. Cl.: C07D 217/20

(54) **PROCESS FOR ASYMMETRIC HYDROGENATION OF HEXAHYDROQUINOLINE SALTS**
VERFAHREN ZUR ASYMMETRISCHEN HYDRIERUNG VON HEXAHYDROCHINOLINSALZEN
PROCEDE D'HYDROGENATION ASYMETRIQUE DE SELS D'HEXAHYDROQUINOLINE

(30) Priority: 19.03.2002 EP 02006124
(43) Date of publication of application: 15.12.2004
(73) Proprietor: DSM IP Assets B.V., 6411 TE Heerlen (NL)
(72) Inventor: PUENTENER, Kurt, CH-4054 Basel (CH); SCALONE, Michelangelo, CH-4127 Birsfelden (CH); WANG, Shaoning, CH-4058 Basel (CH)
(74) Representative: Schwander, Kuno Josef, Dr.
(86) International application number: PCT/EP2003/002610
(87) International publication number: WO 2003/078399

(56) References cited:
- EP-A- 0 850 931
- CULLEN W R ET AL: "Asymmetric homogeneous hydrogenation of imines using rhodium-phosphine systems" JOURNAL OF MOLECULAR CATALYSIS, LAUSANNE, CH, vol. 62, 1990, pages 243-253, XP000864377
- NG CHEONG CHAN Y ET AL: "Iridium (III) Hydride Complexes for the Catalytic Enantioselective Hydrogenation of Imines" JOURNAL OF THE AMERICAN CHEMICAL SOCIETY, AMERICAN CHEMICAL SOCIETY, WASHINGTON, DC, US, vol. 112, 1990, pages 9400-9401, XP000864363 ISSN: 0002-7863
- IMWINKELRIED R: "Catalytic Asymmetric Hydrogenation in the Manufacture of d-Biotin and Dextromethorphan" CHIMIA, AARAU, CH, vol. 51, no. 6, 1997, pages 300-302, XP000864390 ISSN: 0009-4293

## Description

The present invention relates to a process for the preparation of (S) or (R)-1-(4-methoxybenzyl)-1,2,3,4,5,6,7,8-octahydroisoquinolinium salts. More particularly the invention relates to a process for the preparation of (S) or (R)-1-(4-methoxybenzyl)-1,2,3,4,5,6,7,8-octahydroisoquinolium salts by asymmetric hydrogenation of a 1-(4-methoxybenzyl)-3,4,5,6,7,8-hexahydroisoquinolinium salt. (S) -1-(4-methoxybenzyl)-1,2,3,4,5,6,7,8-octahydroisoquinoline and salts thereof are an intermediate product in the manufacture of dextromethorphan, a known antitussive agent.

A process for the asymmetric hydrogenation of 1-(4-methoxybenzyl)-3,4,5,6,7,8-hexahydroisoquinolinium salts in the presence of Ir complexes of chiral diphosphin ligands to yield (S) or (R)-1-(4-methoxybenzyl)-1,2,3,4,5,6,7,8-octa-hydro-isoquinoline and salts thereof, respectively, is known, e.g., from European patent application no. 0 850 931 A.

In accordance with the present invention it has been found that the asymmetric hydrogenation of 1-(4-methoxybenzyl)-3,4,5,6,7,8-hexahydroisoquinolium salts to yield (S) or (R)-1-(4-methoxybenzyl)-1,2,3,4,5,6,7,8-octahydroisoquinolium salts can be effected with superior optical yield by the use of an iridium or rhodium complex catalyst comprising a chiral diphosphine ligand of the general formula I or II 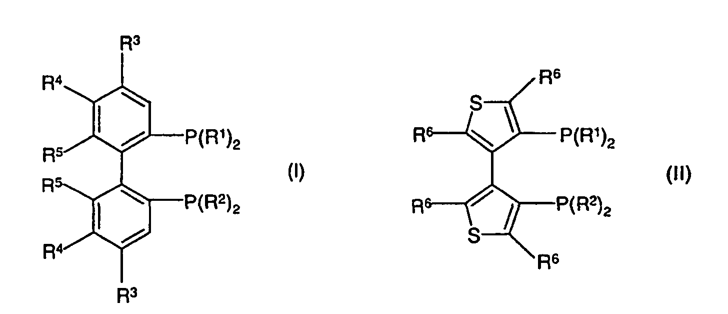 a neutral ligand and an anion;
wherein in the general formulas I and II
R¹ and R² are, independently, phenyl substituted by 2 to 5 C₁₋₈-alkyl, C₁₋₈-alkoxy, di-
(C₁₋₈-alkyl)amino, morpholino, phenyl or tri-C₁₋₈-alkyl-silyl groups;
R³ and R⁴ are hydrogen, C₁₋₈ alkyl, C₁₋₈ alkoxy or C₁₋₈ dialkylamino;
R⁵ is C₁₋₈ alkyl, C₁₋₈ alkoxy, hydroxy or C₁₋₈ akyl-C(O)O-; or
R³ and R⁴, or R⁴ and R⁵, or both residues R⁵, taken together, are -X-(CH2)n-X- wherein X is oxygen or -C(O)O- and n is an integer from 1 to 6; or
R³ and R⁴, or R⁴ and R⁵, together with the carbon atoms to which they are attached form a naphthyl, tetrahydronaphthyl or dibenzofuran ring; and
R⁶ is C₁₋₈ alkyl.

Accordingly, the present invention relates to a process for the preparation of (S) or (R)-1-(4-methoxybenzyl)-1,2,3,4,5,6,7,8-octahydroisoquinolinium salts wherein a 1-(4-methoxybenzyl)-3,4,5,6,7,8-hexahydroisoquinolinium salt is asymmetrically hydrogenated in the presence of a base and an iridium or rhodium complex comprising a chiral diphosphine ligand of the general formulas I or II as defined above, a neutral ligand and an anion, whereupon the (S) or (R)-1-(4-methoxybenzyl)-1,2,3,4,5,6,7,8-octahydroisoquinol-inium salt obtained is isolated as such or in the form of the free base.

The 1-(4-methoxybenzyl)-3,4,5,6,7,8-hexahydro (and 1,2,3,4,5,6,7,8 octahydro)-isoquinolium salts may be salts of mineral acids, such as sulfuric or phosphoric acid, e.g., the hydrogensulfate or dihydrogenphosphate; or hydrohalic acids e,g., the hydro-chloride, hydrobromide or hydroiodide or perchlorate; or salts of organic acids, e.g. aliphatic or aromatic carboxylic or sulfonic acids e.g., the formate, acetate, propionate, oxalate, succinate, maleinate, benzoate, phthalate, picrate, mesylate, benzenesulfonate or tosylate. The hydrogenation of the hydrogensulfate is preferred.

The term "C₁₋₈-alkyl" denotes straight-chain or branched-chain alkyl groups containing up to 8 carbon atoms, such as, for example, methyl, ethyl, propyl, isopropyl, butyl, isobutyl, tert.-butyl, pentyl, isopentyl, neopentyl, hexyl, tert.hexyl, heptyl, isoheptyl, octyl and isooctyl. Similarly, the term term "C₁₋₈-alkoxy" denotes an alkyl group as defined above, which is bonded via an oxygen atom such as methoxy, ethoxy, propoxy, isopropoxy, butoxy and the like.

Preferably, R¹ and R² denote phenyl carrying 2-5, especially two C3-8 branched chain alkyl groups, i.e., alkyl groups containing 3 to 8 carbon atoms of which at least one carbon atom is a tertiary or quaternary carbon atom, i.e. a carbon atom which is bonded to three or four other carbon atoms. Examples of C₃₋₈ branched chain alkyl groups are isopropyl, tert.-butyl, tert.-pentyl and isopentyl.

Preferred ligands of formulas I and II are those wherein R¹ and R² are 3,5-di-tert.butyl-phenyl, 3,5-di-tert.pentyl-phenyl or 3,5-di-tert.butyl-4-methoxyphenyl; R³ and R⁴ are hydrogen; R⁵ is C1-8 alkoxy, particularly methoxy, C₁₋₈ alkyl, particularly methyl, or hydroxy; and R⁶ is methyl. Most preferred are the ligands of formula I especially those wherein R¹ and R² are 3,5-di-tert.butyl-phenyl, R³ and R⁴ are hydrogen, and R⁵ is methoxy. When using ligands of formulas I or II having (S) configuration, (S)-1-(4-methoxybenzyl)-1,2,3,4,5,6,7,8-octahydroisoquinolinium salts are obtained in high enantiomeric purity.

The preferred catalysts for the preparation of (S)-1-(4-methoxybenzyl)-1,2,3,4,5,6,7,8-octahydroisoquinolinium salts by the process of the present invention are, accordingly, those wherein the chiral diphosphine ligand has (S)-configuration and is of the general formula I, wherein R¹ and R² are 3,5-di-tert.butyl-phenyl, R⁵ is methoxy and R³ and R⁴ are hydrogen. Further preferred catalysts are those wherein the central atom is iridium.

The term "neutral ligand" as used herein denotes a readily exchangeable ligand such as an olefin, e.g. ethylene, propylene, cyclooctene, 1,5-hexadiene, norbornadiene, 1,5-cyclooctadiene, benzene, hexamethylbenzene, p-cymene and the like, a nitrile such as acetonitrile or benzonitrile, or also solvent such as e.g. tetrahydrofuran, toluene etc. Where more than one such ligand is present, these can also be different from each other. A preferred neutral ligand is 1,5-cyclooctadiene (COD).

The anion in the catalyst used according to the present invention may be the anion of a hydrohalic acid, such as, for example, Cl⁻, Br⁻, I⁻; an oxygen acid ClO₄⁻, BrO₄⁻, IO₄⁻, NO₃⁻, CF₃COO⁻, CF₃SO₃⁻ or C₆H₅SO₃⁻ as well as halogen or aryl complexes with the elements boron, phosphorus, arsenic, antimony or bismuth, such as BF₄⁻, PF₆⁻ and SbF₆⁻; or B(phenyl)4⁻ and B(3,5-di-trifluoromethyl-phenyl)4⁻ (BARF⁻). Preferred anions are Cl⁻, ClO₄⁻, BF₄⁻ or PF₆⁻ and BARF⁻, especially Cl⁻ and BARF⁻.

The catalysts for use in the process of the present invention may be prepared by reacting a compound [Ir(L)Cl]₂ or [Rh(L)Cl]₂, or [Ir(L⁺)₂]BF₄⁻ or [Rh(L⁺)₂]BF₄⁻,where L denotes a neutral ligand, e.g. COD, with the desired ligand of formula I or II, e.g., ((*S*)-(6,6')-dimethoxybiphenyl-2,2'-diyl)bis[bis(3,5-di-tert.butylphenyl)phosphin] (I, R¹, R² = 3,5-tert.-butylphenyl; R³, R⁴ = H; R⁵ = methoxy, hereinafter: (S)-3,5-tBu-MeOBIPHEP) in an appropriate solvent, such as methanol. The catalyst may be used after isolation or as prepared *in situ*. The ligands of formula I and II can be prepared by methods known per se, e.g. as described in EP 0 398 132 or WO 96/01831, or in analogy thereto. The compounds, [Ir(L)Cl]₂, [Rh(L)Cl]₂, [Ir(L⁺)₂]BF₄⁻ and [Rh(L⁺)₂]BF₄⁻ are either commercially available, e.g., from Strem Chemicals Inc., Newburgport, Mass., USA or Sigma-Aldrich-Fluka, Buchs, SG, Switzerland, or can be prepared by methods known per se, e.g. by reacting the appropriate Ir or Rh salt with the appropriate neutral ligand as described, e.g., in J.Chem.Soc. 1957, 4735, or in Chem. Berichte 1966,99,3610, or in J.Am.Chem.Soc. 1971,93,2397.

The asymmetric hydrogenation in accordance with the present invention is effected in a suitable organic solvent which is inert under the reaction conditions. Suitable solvents are especially lower alcohols, such as methanol, ethanol and isopropanol and mixtures thereof, e.g. mixtures of methanol and isopropanol; and mixtures of methanol with methylene chloride or toluene, e.g. mixtures of methanol/methylene chloride (1:1 by volume), and methanol/toluene (9:1 to 1:2 by volume). Also, methanol containing minor amounts of water, e.g. 1 % (by vol.) of water may be used. Methanol or ethanol based solvents are preferred.

The process in accordance with the invention is carried out in the presence of a base. The base may be a carboxylic acid salt, such as e.g. sodium acetate, sodium formate and the like, a primary, secondary and tertiary amine, such as, for example, diisopropyl-amine, triethylamine, diisopropylethylamine or a-methylbenzylamine. Also, the reaction product, 1-(4-methoxybenzyl)-1,2,3,4,5,6,7,8-octa-hydro-isoquinoline may serve as the base. Tertiary amines, particularly diisopropyl--ethylamine and triethylamine are especially preferred. Further examples of bases for use in the hydrogenation of the present invention are carbonates, such as sodium and potassium hydrogen carbonate, and caesium carbonate; and potassium phosphate; and phenolates, such as sodium or potassium phenolate.

The amount of base used maybe in the range of about 0.01 to about 1 mol equivalent, preferably about 0.01 to about 0.1 mol equivalent per mol of starting material, 1-(4-methoxybenzyl)-3,4,5,6,7,8-hexahydro-isoquinol-inium salt.

The asymmetric hydrogenation in accordance with the present invention is conveniently carried out at temperatures in the range of about 20 °C to about 100 °C, preferably at about 60 °C to 80 °C when using an Ir catalyst, and about 40 °C to 60 °C when using an Rh catalyst. The hydrogen pressure is not narrowly critical and pressures of e.g., about 1 to 100 bar, conveniently 20-50 bar, may be used. While the catalyst may be prepared *in situ*, it has been found that the hydrogenation process is facilitated when the catalyst, especially an Ir catalyst, is added to the reaction mixture to be hydrogenated as a preformed crystalline complex.

The molar ratio of substrate to catalyst (S/C) between the compound to be hydrogen-ated and the catalyst complex comprising the ligand of formula I or II may vary within a broad range, e.g., between about 100 to 100 000, and is suitably between about 10 000 and about 30 000. The concentration of the substrate in the reaction mixture is not narrowly critical and may conveniently range between about 5 wt.-% and about 50 wt.-%.

The octahydroisoquinolinium salt obtained may be isolated from the reaction mixture as such or may be converted into the base, i.e. the octahydroisoquinoline, by treatment with the appropriate amount of a base such as an alkali hydroxide, e.g. sodium hydroxide, and isolated in that form by extraction with a solvent, e.g., hexane.

The invention is illustrated further by the Examples which follow.

### Example 1

### a) In-situ preparation of the catalyst solution

In a glove box a 50 ml Erlenmeyer flask was charged with 7.13 mg (1.06×10⁻⁵ mol) of [Ir(COD)Cl]₂ , 24.08 mg (2.33×10⁻⁵ mol) of (S)-3,5-tBu-MeOBIPHEP and 30 ml of methanol. The resulting yellow orange solution was stirred for 30 min at room tempera-ture. The stirring bar was removed and the orange solution was transferred with aid of 10 ml of methanol to a 200-ml catalyst addition tube, which was then sealed, pressurized with 7 bar of argon, removed from the glove box and finally connected to an autoclave.

### b) Asymmetric hydrogenation

The autoclave was charged with 150.0 g of 1-(4-methoxybenzyl)-3,4,5,6,7,8-hexahydroisoquinoline hydrogensulfate, 720 ml of methanol and 0.73 ml of N-ethyldiisopropylamine. The autoclave was sealed and inertized under stirring by 4 cycles of evacuation to 0.1 bar and pressurizing with 2 bar of argon. Afterwards, the pressure was reduced to 1 bar. Then, the catalyst solution was introduced into the autoclave from the catalyst addition tube. The autoclave was then connected to a hydrogen line, which was thoroughly flushed with hydrogen. Under stirring the residual argon and oxygen were removed by pressurizing the autoclave with 10 bar of hydrogen for 2 min and reducing the pressure to 2 bar (4 cycles). Finally, the hydrogen pressure was set at 30 bar and the heating of the contents of the autoclave started. After 45 min the reaction temperature of 80°C was reached while the pressure slightly increased to 31 bar. The hydrogen pressure was now set to constant 35 bar. After 10 h the autoclave was cooled to 20°C within 1 h. The hydrogen pressure was released and the autoclave inertized by 3 cycles of evacuation to 0.5 bar and pressurizing with 2 bar of argon (3 cycles). The slightly yellow solution was transferred to a 2-1 round-bottomed flask and the autoclave was washed three times with 300 ml, a total of 900 ml of methanol. The combined methanolic solutions were evaporated to dryness (70°C/70mbar).

### c) Isolation of the hydrogenation product

The oily residue (170.2 g) from the above hydrogenation reaction was dissolved in 400 ml of warm water and extracted, after addition of 30 ml of 2N sulfuric acid with 200 ml of t-butyl methylether. The organic layer was washed with 100 ml of water and evaporated to dryness. To the combined aqueous phase 136 g of 28% NaOH and 400 ml of hexane were added with stirring. The biphasic mixture was transferred with aid of 200 ml of hexane in a separation funnel. The aqueous layer was separated and extracted with 400 ml of hexane. The combined organic phases were washed successively 4 times with 100 ml, a total of 400 ml of water. The combined organic layers were rotatory evaporated (60°C/40mbar) and the residue dried (20°C/9 mbar) for 1 h to yield 106.4 g (99 %) of crude (S)-1-(4-methoxybenzyl)-1,2,3,4,5,6,7,8--octahydro--isoquinoline as an orange oil in a chemical purity of 99.1% and an enantiomeric purity of of 84.7 %.

### Example 2

### a) In-situ preparation of the catalyst solution

In a glove box a 50 ml Erlenmeyer flask was charged with 6.98 mg (1.42×10⁻⁵ mol) of [Rh(COD)Cl]₂, 32.10 mg (3.11×10⁻⁵ mol) of (S)-3,5-tBu-MeOBIPHEP and 30 ml of ethanol. The resulting yellow orange solution was stirred for 30 min at room tempera-ture. 5.9 ml of triethylamine were added and the solution stirred for additional 5 minutes. The orange solution was transferred with aid of 10 ml of ethanol to a 200-ml catalyst addition tube, which was sealed, pressurized with 7 bar of argon, removed from the glove box and finally connected to an autoclave.

### b) Asymmetric hydrogenation

An autoclave was charged with 150.0 g of 1-(4-methoxybenzyl)-3,4,5,6,7,8-hexahydroisoquinoline hydrogensulfate and 720 ml of ethanol. The autoclave was sealed, inertized under stirring by 8 cycles of evacuation to 0.1 bar and pressurizing with 2 bar of argon. The reaction mixture was then heated with stirring. After 30 min the reaction temperature of 60°C was reached while the pressure slightly increased to 2.8 bar. To remove residual oxygen the autoclave was carefully evaporated to 0.1 bar and pressurized with 2 bar of argon (two cycles). Then the autoclave was connected to a hydrogen line, which was thoroughly flushed with hydrogen. Under stirring the residual argon and oxygen were removed by pressurizing the autoclave with 10 bar of hydrogen for 2 min and reducing the pressure to 2 bar (2 cycles). Then, the bottom valve of the catalyst addition device was opened and the catalyst solution introduced into the autoclave. The hydrogen pressure was now set to constant 35 bar. After 18 h the autoclave was cooled to 20°C within 1 h. The hydrogen pressure was released and the autoclave inertized by 3 cycles of evacuation to 0.5 bar and pressurizing with 2 bar of argon (3 cycles). The slightly yellow solution was transferred to a 2-1 round-bottomed flask and the autoclave was washed with three times with 300 ml, a total of 900 ml of methanol. The combined alcoholic solutions were evaporated to dryness (70°C/50mbar).

### c) Isolation of the hydrogenation product

The oily residue (174.0 g) from the above hydrogenation reaction was dissolved in 400 ml of warm water and extracted, after addition of 30 ml of 2N sulfuric acid with 200 ml of t-butyl methylether The organic layer was washed with 100 ml of water and evaporated to dryness. To the combined aqueous phases 136 g of 28% NaOH and 400 ml of hexane were added. The biphasic mixture was transferred with aid of 200 ml of hexane in a 2-1 separation funnel. The aqueous layer was separated, transferred into a second 2-1 separation funnel and extracted with 400 ml of hexane. The organic phases in both separation funnels were washed successively 4 times with 100 ml, a total of 400 ml of water. The combined organic layers were rotatory evaporated (60°C/40mbar) and the residue dried (20°C/9 mbar) for 1 h to yield 105.0 g (98.4 %) of crude (S)-1-(4-methoxybenzyl)-1,2,3,4,5,6,7,8--octahydro--isoquinoline as an orange oil of a chemical purity of 98.4% and an enantiomeric purity (ee) of 91.1 %.

### Example 3

In analogy to Example 1 the hydrogenation was carried out applying the following process conditions:
Catalyst: [Ir((S)-3,5-tBu-MeOBIPHEP)(COD)]Cl ;substrate/catalyst molar ratio 20.000; 0.01 eq. diisopropyl-ethylamine rel. to hexahydroisoquinoline hydrogensulfate starting
material; 35 bar H2; 80 °C; c= 20 wt.-% in methanol. After 5 h reaction time, the conversion was 99.7 %, selectivity was 99.1 %, and enantiomeric excess (ee) was 85 %.

### Example 4

In analogy to Example 3 but using [Ir((S)-3,5-tBu-MeOBIPHEP)(COD)]BARF^ as the catalyst and a reaction time of 6 h, the conversion was 99.8 %, selectivity was 99.4 %, and enantiomeric excess (ee) was 84 %.

### Example 5

In analogy to Example 4 but using 0.1 eq. of triethylamine; a substrate/catalyst molar ratio of 10.000; 40 bar H2; 80 °C; c= 10 wt.-% in methanol and a reaction time of 15 h, the conversion was >99.9 %, selectivity was 99 %, and enantiomeric excess (ee) was 83 %.

### Example 6

In analogy to Example 5 but using 0.1 eq. of Cs₂CO₃ as the base the conversion was 99.8 %, selectivity was 98 %, and enantiomeric excess (ee) was 83 %.

### Example 7

In analogy to Example 5 but using 0.1 eq. of sodium acetate as the base the conversion was 99.6 %, selectivity was 98 %, and enantiomeric excess (ee) was 83 %.

### Example 8

In analogy to Example 2 the hydrogenation was carried out applying the following process conditions:
Catalyst: [Rh(COD) Cl] 2 / (S)-3,5-tBu-MeOBIPHEP (1:1.1 mol);substrate/catalyst molar ratio 10.000; 0.1 eq. diisopropyl-ethylamine rel. to hexahydroisoquinoline hydrogensulfate starting material; 40 bar H₂; 60 °C; c= 10 wt.-% in ethanol. After 15 h reaction time, the conversion was >99.9 %, selectivity was 99 %, and enantiomeric excess (ee) was 91 %.

### Example 9

In analogy to Example 8 but using [Rh(COD)₂]BARF⁻ for the catalyst the conversion was >99.9 %, selectivity was 99 %, and enantiomeric excess (ee) was 91 %.

### Example 10

In analogy to Example 8 but using [Rh(COD)₂]ClO₄ or [Rh(COD)₂]BF₄ for the catalyst the conversion was 99.9 %, selectivity was 99 %, and enantiomeric excess (ee) was 91 %.

### Example 11

In analogy to Example 8 but using [Rh(COD)₂]PF₆ for the catalyst the conversion was 99.7 %, selectivity was 99 %, and enantiomeric excess (ee) was 91 %.

### Example 12

In analogy to Example 8 but using a [Rh(COD)Cl] ₂/ (S)-3,5-tBu-MeOBIPHEP ratio of 1 : 1.3; 0.1 eq. of triethylamine and c= 20 wt.-% in ethanol, the conversion after 4 h reaction time was 99 %, selectivity was 99.5 %, and enantiomeric excess (ee) was 91.4 %.

### Example 13

In analogy to Example 12 but using a [Rh(COD)Cl]₂/(S)-3,5-tBu-MeOBIPHEP ratio of 1 : 1.1; the conversion after 4 h reaction time was 99 %, selectivity was 99.4 %, and enantiomeric excess (ee) was 91.3 %.

### Example 14

In analogy to Example 2 but using a[Rh(COD)Cl]₂/(S)-3,5-tBu-MeOBIPHEP ratio of 0,5:1.1 mol; a substrate/catalyst molar ratio 10.000; 0.1 eq. triethylamine relative to hexahydroisoquinoline hydrogensulfate starting material; 35 bar H₂; 40 °C; c= 20 wt.-% in ethanol there was obtained, after 15 h reaction time, a conversion of >99.9 %, a selectivity of >99.9 %, and an enantiomeric excess (ee) of 94 %.

### Example 15

In analogy to Example 1 but using (S)-(6,6'-O-[1,3-propylene]-oxybiphenyl-2,2'-diyl) bis(di(3,5-di-tert.-butylphenyl)phosphine as chiral diphosphine ligand, the conversion after 10 h was >99.9 %, selectivity was 97 %, and enantiomeric excess (ee) was 81 %.

### Example 16

In analogy to Example 12 but using (S)-(6,6'-O-[1,3-butylene]-oxybiphenyl-2,2'-diyl) bis(di(3,5-di-tert.-butylphenyl)phosphine as chiral diphosphine ligand and c= 10 wt.-% in ethanol, the conversion after 15 h was >99.9 %, selectivity was 99 %, and enantiomeric excess (ee) was 84 %.

### Example 17

### a) Peparation of the pre-formed crystalline catalyst: [Ir((S)-3,5-tBu-MeOBIPHEP)(COD)]BARF

To 300 mg (0.291 mmol) of (S)-3,5-tBu-MeOBIPHEP and 0.389 mg (0.305 mmol) of [Ir(COD)₂]BARF there were added 10 ml of toluene and 10 ml of methanol. The solution was stirred for 1 hour at room temperature, evaporated to dryness and the residue dried under high vacuum at room temperature. The residue was washed 2 times with 20 ml of pentane each under Ar and dried under high vacuum at room temperature. There were obained 0.572 g (90%) of catalyst, [Ir((S)-3,5-tBu-MeOBIPHEP)(COD)]BARF in crystalline form.
³¹P-NMR (CDCl₃): 18.1 ppm (singlett)

### b) Asymmetric hydrogenation

An autoclave of 380 ml was charged with 20 g (55.06 mmol) of 1-(4-methoxybenzyl)-3,4,5,6,7,8-hexahydroisoquinoline hydrogensulfate (97.3 % pure), 12.41 mg of catalyst obtained in paragraph a), and 100 ml of methanol. Then, 0.96 ml of N-ethyldiisopropylamine (98 % pure) were added. All operations were carried out under admission of air. The autoclave was connected to a hydrogen line which was thoroughly flushed with hydrogen. The autoclave was then pressurized and flushed with hydrogen under 40 bar while stirring. Eventually, the hydrogen pressure was set to 40 bar. The autoclave was heated to 80 °C and the hydrogenation allowed to proceed for 15 hours. The autoclave was the cooled and vented and the slightly yellow solution transferred with the aid of 10 ml of methanol to a 250-ml round-bottomed flask. The solution was evaporated to dryness (54 °C/40 mbar).

### c) Isolation of the hydrogenation product

The oily residue (21.1 g) from the above hydrogenation reaction was worked up as described in Example 1, to afford 14.27 g of (S)-1-(4-methoxybenzyl)-1,2,3,4,5,6,7,8-octahydroisoquinoline as a slightly yellow oil in a chemical purity of 97.9 %; enantiomeric purity ee was 83.5%.

## Claims

1. A process for the preparation of (S) or (R)-1-(4-methoxybenzyl)-1,2,3,4,5,6,7,8-octahydroisoquinoline and salts thereof wherein a 1-(4-methoxybenzyl)- 3,4,5,6,7,8-hexahydroisoquinolinium salt is asymmetrically hydrogenated in the presence of a base and an iridium or rhodium complex comprising a chiral diphosphine ligand of the general formula I or II, 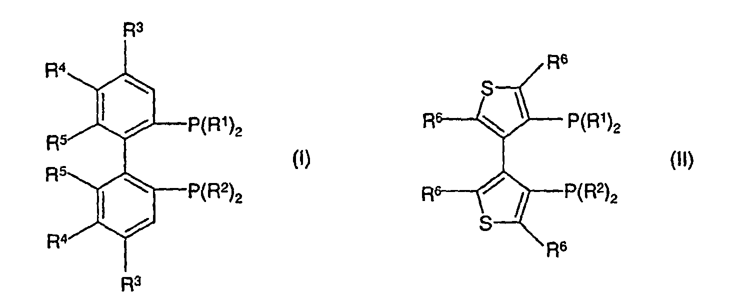 a neutral ligand and an anion;
wherein in the general formula I and II
R¹ and R² are, independently, phenyl substituted by 2 to 5 C₁₋₈-alkyl, C₁₋₈-alkoxy, di-
(C₁₋₈-alkyl)amino, morpholino, phenyl or tri-C₁₋₈-alkyl-silyl groups;
R³ and R⁴ are hydrogen, C₁₋₈ alkyl, C₁₋₈ alkoxy or C₁₋₈ dialkylamino;
R⁵ is C₁₋₈ alkyl, C₁₋₈ alkoxy, hydroxy or C₁₋₈ alkyl-C(O)O-; or
R³ and R⁴, or R⁴ and R⁵, or both residues R⁵, taken together, are -X-(CH₂)n-X- wherein
X is oxygen or -C(O)O- and n is an integer from 1 to 6; or
R³ and R⁴, or R⁴ and R⁵, together with the carbon atoms to which they are attached form a naphthyl, tetrahydronaphthyl or dibenzofuran ring; and
R⁶ is C₁₋₈ alkyl;
whereupon the (S) or (R)-1-(4-methoxybenzyl)-1,2,3,4,5,6,7,8-octahydroisoquinol-inium salt obtained is isolated as such or in the form of the free base.

2. A process as in claim 1 wherein the chiral diphosphine ligand is of the general formula I.

3. A process as in claim 2 wherein in the chiral diphosphine ligand R¹ and R² are 3,5-di-tert.butyl-phenyl, 3,5-di-tert.pentyl-phenyl or 3,5-di-tert.butyl-4-methoxyphenyl.

4. A process as in claim 3 wherein in the chiral diphosphine ligand R¹ and R² are 3,5-di-tert. butyl-phenyl.

5. A process as in any one of claims 2 to 4 wherein in the chiral diphosphine ligand R⁵ is methoxy and R³ and R⁴ are hydrogen.

6. A process as in any one of claims 2 to 5 wherein the base is di-(isopropyl)ethylamine.

7. A process as in any one of claims 1 to 5 wherein about 0.01 to about 0.1 mol equivalent of base per mol of 1-(4-methoxybenzyl)-3,4,5,6,7,8-hexahydro-isoquinol-inium salt is used.

8. A process as in any one of claims 1 to 7 wherein the neutral ligand is 1,5-cyclooctadiene.

9. A process as in any one of claims 1 to 8, wherein the anion is Cl⁻ or BARF⁻.

10. A process as in any one of claims 1 to 9 wherein the complex is an iridium complex.

11. A process as in any one of claims 1 to 10 wherein the catalyst is added to the reaction mixture to be hydrogenated as a pre-formed crystalline complex under admission of air.

12. A process as in any one of claims 1 to 11 wherein methanol or ethanol are used as a solvent.

13. A process as in any one of claims 1 to 12 wherein a catalyst comprising a chiral diphosphine ligand of the general formula I having (S) configuration is used to produce (S)-1-(4-methoxybenzyl)-1,2,3,4,5,6,7,8-octahydroisoquinoline.

## Patentansprüche

1. Verfahren zur Herstellung von (S)- oder (R)-1-(4-Methoxybenzyl)-1,2,3,4,5,6,7,8-octahydroisochinolin und Salzen davon, wobei ein 1-(4-Methoxybenzyl)-3,4,5,6,7,8-hexahydroisochinoliniumsalz in Gegenwart einer Base und eines Iridium- oder Rhodiumkomplexes, umfassend einen chiralen Disphosphinliganden der allgemeinen Formel I oder II 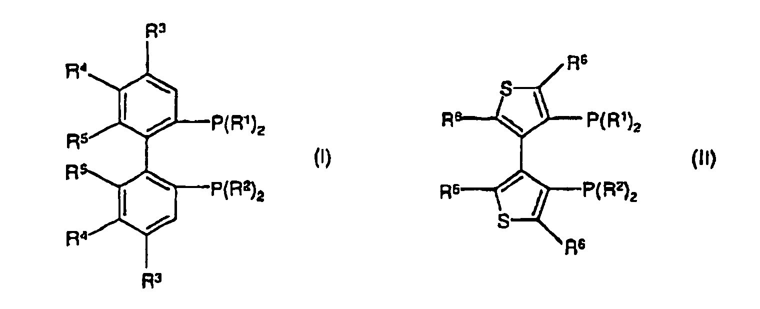 einen neutralen Liganden und ein Anion, asymmetrisch hydriert wird;
wobei in der allgemeinen Formel I und II
R¹ und R² unabhängig Phenyl sind, substituiert durch 2 bis 5 C₁₋₈-Alkyl-, C₁₋₈-Alkoxy-, Di-(C₁₋₈-alkyl)amino-, Morpholino-, Phenyl- oder Tri-C₁₋₈-alkylsilyl-Gruppen;
R³ und R⁴ Wasserstoff, C₁₋₈-Alkyl, C₁₋₈-Alkoxy oder C₁₋₈-Dialkylamino sind,
R⁵ C₁₋₈-Alkyl, C₁₋₈-Alkoxy, Hydroxy oder C₁₋₈-Alkyl-C(O)O- ist; oder
R³ und R⁴ oder R⁴ und R⁵ oder beide Reste R⁵ zusammengenommen -X-(CH₂)ₙ-X- sind, wobei
X Sauerstoff oder -C(O)O- ist und n eine ganze Zahl von 1 bis 6 ist; oder
R³ und R⁴ oder R⁴ und R⁵ zusammen mit den Kohlenstoffatomen, an die sie gebunden sind, einen Naphthyl-, Tetrahydronaphthyl- oder Dibenzofuranring bilden; und
R⁶ C₁₋₈-Alkyl ist;
woraufhin das erhaltene (S)- oder (R)-1-(4-Methoxybenzyl)-1,2,3,4,5,6,7,8-octahydroisochinoliniumsalz als solches oder in Form der freien Base isoliert wird.

2. Verfahren nach Anspruch 1, wobei der chirale Diphosphinligand die allgemeine Formel I aufweist.

3. Verfahren nach Anspruch 2, wobei in dem chiralen Diphosphinligand R¹ und R² 3,5-Di-tert.butyl-phenyl, 3,5-Di-tert.pentyl-phenyl oder 3,5-Di-tert.butyl-4-methoxyphenyl sind.

4. Verfahren nach Anspruch 3, wobei in dem chiralen Diphosphinligand R¹ und R² 3,5-Di-tert.butyl-phenyl sind.

5. Verfahren nach einem der Ansprüche 2 bis 4, wobei in dem chiralen Diphosphinligand R⁵ Methoxy ist und R³ und R⁴ Wasserstoff sind.

6. Verfahren nach einem der Ansprüche 2 bis 5, wobei die Base Di-(isopropyl)ethylamin ist.

7. Verfahren nach einem der Ansprüche 1 bis 5, wobei etwa 0,01 bis etwa 0,1 Moläquivalent der Base pro Mol 1-(4-Methoxybenzyl)-3,4,5,6,7,8-hexahydroisochinoliniumsalz verwendet wird.

8. Verfahren nach einem der Ansprüche 1 bis 7, wobei der neutrale Ligand 1,5-Cyclooctadien ist.

9. Verfahren nach einem der Ansprüche 1 bis 8, wobei das Anion Cl⁻ oder BARF⁻ ist.

10. Verfahren nach einem der Ansprüche 1 bis 9, wobei der Komplex ein Iridiumkomplex ist.

11. Verfahren nach einem der Ansprüche 1 bis 10, wobei der Katalysator zu dem Reaktionsgemisch, das hydriert werden soll, als ein vorgeformter kristalliner Komplex unter Zufuhr von Luft zugegeben wird.

12. Verfahren nach einem der Ansprüche 1 bis 11, wobei Methanol oder Ethanol als ein Lösungsmittel verwendet werden.

13. Verfahren nach einem der Ansprüche 1 bis 12, wobei ein Katalysator, umfassend einen chiralen Diphosphinliganden der allgemeinen Formel I mit (S)-Konfiguration, verwendet wird, um (S)-1-(4-Methoxybenzyl)-1,2,3,4,5,6,7,8-octahydroisochinolin herzustellen.

## Revendications

1. Procédé de préparation de (S)- ou (R)-1-(4-méthoxybenzyl)-1,2,3,4,5,6,7,8-octahydroisoquinoléine et de ses sels, dans lequel on soumet un sel de 1-(4-méthoxybenzyl)-3,4,5,6,7,8-hexahydroisoquinolinium à une hydrogénation asymétrique en présence d'une base et d'un complexe d'iridium ou de rhodium comprenant un ligand diphosphine chirale de formule générale I ou II, 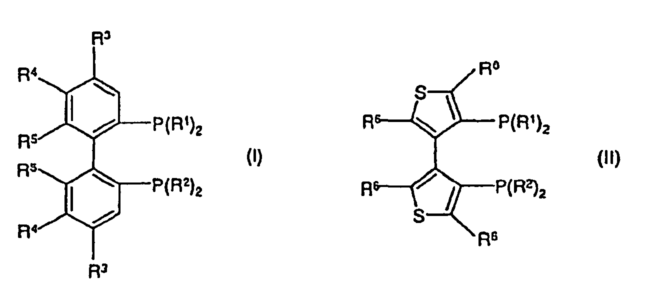 un ligand neutre et un anion ;
où, dans les formules générales I et II,
R¹ et R² représentent, indépendamment l'un de l'autre, un groupe phényle substitué par 2 à 5 groupes alkyle en C₁ à C₈, alcoxy en C₁ à C₈, di(alkyle en C₁ à C₈)amino, morpholino, phényle ou tri(alkyle en C₁ à C₈)silyle ;
R³ et R⁴ représentent un atome d'hydrogène, un groupe alkyle en C₁ à C₈, alcoxy en C₁ à C₈ ou di(alkyle en C₁ à C₈)amino ;
R⁵ représente un groupe alkyle en C₁ à C₈, alcoxy en C₁ à C₈, hydroxy ou (alkyle en C₁ à C₈)-C(O)O- ; ou
R³ et R⁴, ou R⁴ et R⁵, ou les deux groupes R⁵, pris ensemble, forment un groupe -X-(CH₂)n-X- dans lequel X représente un atome d'oxygène ou -C(O)O- et n est un nombre entier de 1 à 6 ; ou
R³ et R⁴, ou R⁴ et R⁵, conjointement avec les atomes de carbone auxquels ils sont liés, forment un noyau naphtyle, tétrahydronaphtyle ou dibenzofuranne ; et
R⁶ représente un groupe alkyle en C₁ à C₈ ;
après quoi on isole le sel de (S)- ou (R)-1-(4-méthoxybenzyl)-1,2,3,4,5,6,7,8-octahydroisoquinolinium tel quel ou sous la forme de la base libre.

2. Procédé selon la revendication 1, dans lequel le ligand diphosphine chirale est de formule générale I.

3. Procédé selon la revendication 2, dans lequel, dans le ligand diphosphine chirale, R¹ et R² représentent un groupe 3,5-di-tert-butyl-phényle, 3,5-di-tert-pentylphényle ou 3,5-di-tert-butyl-4-méthoxyphényle.

4. Procédé selon la revendication 3, dans lequel, dans le ligand diphosphine chirale, R¹ et R² représentent un groupe 3,5-di-tert-butyl-phényle.

5. Procédé selon l'une quelconque des revendications 2 à 4, dans lequel, dans le ligand diphosphine chirale, R⁵ représente un groupe méthoxy et R³ et R⁴ représentent un atome d'hydrogène.

6. Procédé selon l'une quelconque des revendications 2 à 5, dans lequel la base est la di-(isopropyl)éthylamine.

7. Procédé selon l'une quelconque des revendications 1 à 5, dans lequel environ 0,01 à environ 0,1 équivalent molaire de base par mole de sel de 1-(4-méthoxybenzyl)-3,4,5,6,7,8-hexahydro-isoquinolinium est utilisé.

8. Procédé selon l'une quelconque des revendications 1 à 7, dans lequel le ligand neutre est le 1,5-cyclooctadiène.

9. Procédé selon l'une quelconque des revendications 1 à 8, dans lequel l'anion est Cl⁻ ou BARF⁻.

10. Procédé selon l'une quelconque des revendications 1 à 9, dans lequel le complexe est un complexe d'iridium.

11. Procédé selon l'une quelconque des revendications 1 à 10, dans lequel le catalyseur est ajouté sous forme de complexe cristallin préformé au mélange réactionnel à hydrogéner sous admission d'air.

12. Procédé selon l'une quelconque des revendications 1 à 11, dans lequel le méthanol ou l'éthanol est utilisé en tant que solvant.

13. Procédé selon l'une quelconque des revendications 1 à 12, dans lequel un catalyseur comprenant un ligand diphosphine chirale de formule générale I ayant une configuration (S) est utilisé pour produire la (S)-1-(4-méthoxybenzyl)-1,2,3,4,5,6,7,8-octahydroisoquinoléine.
